# EUROPÄISCHE PATENTSCHRIFT

(11) **EP 3 350 081 B1**
(45) Veröffentlichungstag und Bekanntmachung des Hinweises auf die Patenterteilung: **19.06.2019**
(21) Anmeldenummer: 16766944.9
(22) Anmeldetag: 15.09.2016
(51) Int. Cl.: B65B 3/00

(54) **VORRICHTUNG UND VERFAHREN ZUM BEFÜLLEN EINER ELEKTRISCHEN ZIGARETTE**
DEVICE AND METHOD FOR FILLING AN ELECTRIC CIGARETTE
DISPOSITIF ET PROCÉDÉ DE REMPLISSAGE D'UNE CIGARETTE ÉLECTRIQUE

(30) Priorität: 15.09.2015 DE 102015217671
(43) Veröffentlichungstag der Anmeldung: 25.07.2018
(73) Patentinhaber: Fontem Holdings 1 B.V., 1083 HN Amsterdam (NL)
(72) Erfinder: BEER, Andreas, 85630 Grasbrunn (DE)
(74) Vertreter: Gulde & Partner
(86) Internationale Anmeldenummer: PCT/EP2016/071829
(87) Internationale Veröffentlichungsnummer: WO 2017/046247

(56) Entgegenhaltungen:
- WO-A1-2011/095781
- WO-A1-2012/070107
- WO-A1-2014/155092

## Beschreibung

Die Erfindung betrifft eine Vorrichtung zum Befüllen einer elektrischen Zigarette, einen Verbrauchsmitteltank einer elektrischen Zigarette, sowie ein Verfahren zum Befüllen eines Verbrauchsmitteltanks einer elektrischen Zigarette.

Elektrische Zigaretten gibt es als Einwegprodukte, die nach Verbrauch des Verbrauchsmittels, des sog. Liquids, entsorgt werden, oder als Mehrwegprodukte, bei denen das Verbrauchsmittel in einen Tank nachgefüllt werden kann. Das Nachfüllen einer elektrischen Zigarette mit Verbrauchsmittel geschieht bekanntermaßen dadurch, dass das Gehäuse der Zigarette aufgeschraubt werden muss, um an den Verbrauchsmitteltank zu gelangen. Das Verbrauchsmittel wird daraufhin mittels einer Pipette oder vergleichbarer Vorrichtungen in die dadurch offene Seite des Zigarettengehäuses geträufelt. Ein solcher Nachfüllvorgang gestaltet sich für den Benutzer wenig komfortabel, denn es wird ihm Geschick und Präzision abverlangt. Des Weiteren ist es möglich, dass Verbrauchsmittel vergossen oder neben die Öffnung geträufelt wird, und in Kontakt mit der Haut des Benutzers gerät. Da das Verbrauchsmittel gewöhnlich Nikotin enthält, welches in solch konzentrierter Form nicht mit der menschlichen Haut in Berührung kommen sollte, besteht ein gewisses Gesundheitsrisiko.

Des Weiteren ist es beim herkömmlichen Befüllvorgang möglich, dass Verbrauchsmittel in Bereiche innerhalb der Zigarette gelangt (eingeträufelt wird), in die es keinesfalls gelangen sollte. Denn ein fehlgeleitetes Verbrauchsmittel kann beim ersten Benutzen der elektrischen Zigarette nach dem Nachfüllen unverdampft, also in flüssiger Form, in Mund und Lunge eines Benutzers geraten. Insbesondere hier besteht ein großes gesundheitliches Risiko, da das Verbrauchsmittel mit hoher Nikotinkonzentration nicht auf die Schleimhäute eines Rauchers gelangen sollte.

Falls eine elektrische Zigarette oder ein herkömmliches Fläschchen mit Verbrauchsmittel in Kinderhände geraten sollte, besteht insbesondere für das Kind ein gesundheitliches Risiko. Denn durch Aufschrauben der Verbrauchsmittelflasche oder durch Aufschrauben der elektrischen Zigarette kann problemlos Verbrauchsmittel entweichen und auf oder in den Kinderkörper gelangen.

WO 2014/155092 A1 offenbart einen unter Druck stehenden Behälter mit einem Reservoir für die Befüllung einer elektronischen Zigarette.

WO 2012/070107 A1 offenbart ein Werkzeug für die Befüllung einer elektronischen Zigarette.

WO 2011/095781 A1 offenbart eine Simulationszigarette und ein mit dieser zusammenwirkendes Behältnis.

Es ist eine Aufgabe der vorliegenden Erfindung, eine Vorrichtung zum Befüllen einer elektrischen Zigarette, einen Verbrauchsmitteltank einer elektrischen Zigarette, sowie ein Verfahren zum Befüllen eines Verbrauchsmitteltanks einer elektrischen Zigarette vorzusehen, wobei der Befüllvorgang leckagefrei und/oder kindersicher ausführbar ist.

Diese Aufgabe wird mittels einer Vorrichtung gemäß Anspruch 1, einem Verbrauchsmitteltank gemäß Anspruch 16, einem System gemäß Anspruch 18 und 19, sowie einem Verfahren gemäß Anspruch 20 und 22 gelöst.

Vorteilhafte Weiterbildungen sind Gegenstand der Unteransprüche.

Die Vorrichtung weist einen ersten Tank als Speicherraum zum Aufnehmen eines Verbrauchsmittels und einen zweiten Tank als Speicherraum zum Aufnehmen eines Ausgleichsfluids, insbesondere von Luft, auf. Mittels einer Betätigungsvorrichtung sind die Volumina der Tanks gegenläufig veränderbar. Dies bedeutet, dass mittels der Betätigungsvorrichtung das Volumen des einen Tanks vergrößerbar ist, und gleichzeitig das Volumen des anderen Tanks verkleinert wird. Durch das Vorsehen eines ersten und eines zweiten Anschlusses, wobei diese jeweils mit einem der Tanks verbunden sind, kann die Vorrichtung an eine zu befüllende elektrische Zigarette angeschlossen werden. Mittels der erfindungsgemäßen Ausgestaltung ist es mittels der Betätigungsvorrichtung möglich, das in dem ersten Tank enthaltene Fluid in die elektrische Zigarette einzuleiten, und gleichzeitig das durch das eingeleitete Verbrauchsmittel verdrängte Luftvolumen aus der elektrischen Zigarette abzusaugen. Dadurch wird der Befüllvorgang einer elektrischen Zigarette vereinfacht und ist leckagefrei ausführbar, da verhindert wird, dass durch befüllungsbedingten Überdruck in dem Verbrauchsmitteltank der elektrischen Zigarette das Verbrauchsmittel an unerwünschter Stelle austritt. Es besteht kein Risiko, dass Verbrauchsmittel ins Freie gelangt und auf Haut oder Schleimhäute eines Benutzers gerät.

Durch das vorzugsweise Vorsehen von zwei getrennten Zylindern zum Ausgestalten des ersten und des zweiten Tanks ist die Abdichtproblematik besonders einfach zu lösen.

Durch das vorzugsweise Vorsehen einer Zahnstange oder anderen Stange mit zahnartigen Eingriffselementen und einer Betätigungsvorrichtung mit Zahnradabschnitt ergibt sich des Weiteren ein niedriger Entwicklungsaufwand zum Ausgestalten der Erfindungsidee.

Durch das vorzugsweise Ausgestalten der Vorrichtung auf solche Art, dass die Volumenänderungen ΔV₁, ΔV₂ der beiden Zylinder ungleichgroß ist, kann eine Konfiguration erzielt werden, in der ein größeres Luftvolumen aus der elektrischen Zigarette während des Befüllvorgangs abgesaugt wird, als Verbrauchsmittel in die Vorrichtung eingeleitet wird. Dadurch wird erreicht, dass durch ein in der elektrischen Zigarette erzeugter Unterdruck das eingeleitete Verbrauchsmittel keinesfalls in einen Bereich innerhalb der elektrischen Zigarette gerät, in dem das Risiko besteht, dass das Verbrauchsmittel beim ersten Benutzen nach dem Befüllen, am Verdampfer vorbei, in Mund und Lunge des Rauchers gerät. Denn bevor das Verbrauchsmittel in solche Bereiche der Zigarette geraten würde, wird es aufgrund des ungleichen ΔV wieder in die Vorrichtung zurückgesaugt, bevor es sich innerhalb der elektrischen Zigarette ausbreiten kann.

Durch das vorzugsweise Vorsehen jeweils einer Kanüle am ersten und am zweiten Anschluss kann eine kostengünstige Ausgestaltung erreicht werden. Denn Kanülen, von beispielsweise einem Durchmesser von 1 mm, sind unter anderem zur medizinischen Anwendung weit verbreitet und können kostengünstig erworben werden.

Durch das vorzugsweise Vorsehen einer Kindersicherung ist es möglich, dass die Betätigungsvorrichtung nur dann bedient werden kann, wenn die gesamte Vorrichtung (zum Befüllen einer elektrischen Zigarette) auch an eine solche elektrische Zigarette angeschlossen ist. Dadurch wird gewährleistet, dass die Betätigungsvorrichtung nicht bedient werden kann, wenn sich die Vorrichtung zum Befüllen der elektrischen Zigarette nicht in einer angeschlossenen Position an einer solchen Zigarette befindet. Wenn keine Kindersicherung vorgesehen ist, könnte durch Bedienen der Betätigungsvorrichtung jederzeit Verbrauchsmittel aus der Vorrichtung ausgestoßen werden und auf Kinderhände gelangen.

Zum Abdichten des Verbrauchsmitteltanks weist vorzugsweise jeder der Anschlüsse wenigstens eine Membran oder ein Schnabelventil oder ein Kugelventil auf. Dabei ist die Verwendung einer Membran eine besonders kostengünstige Lösung. Bei der Verwendung eines Schnabelventils besteht der Vorteil, dass beim Herausziehen der Kanüle aus dem Schnabelventil überschüssiges Verbrauchsmittel, das sich als Tropfen an der Kanüle befindet, in den Verbrauchsmitteltank hinein abgestreift wird. Dadurch verringert sich das Risiko von austretendem Verbrauchsmittel.

Im Folgenden werden mehrere Ausführungsbeispiele der Erfindung anhand der Figuren erläutert. Die folgende Beschreibung stellt keine Beschränkung der Erfindung auf die genannten Ausführungsbeispiele dar. Für den Fachmann ist offensichtlich, dass innerhalb des durch die Ansprüche definierten Schutzbereichs weitere Ausführungsbeispiele möglich sind, die beispielsweise einzelne oder mehrere Merkmale der folgend aufgeführten Ausführungsbeispiele kombinieren, oder gar gänzlich andere Implementierungswege beschreiten.
- Fig. 1a: zeigt eine Schnittansicht eines Ausführungsbeispiels der Vorrichtung 1 mit zwei separaten Zylindern 111, 121, wobei Kolben 112, 122 durch eine Zahnstangen 161 verbunden sind.
- Fig. 1b: zeigt eine Detailansicht des Ausführungsbeispiels aus Fig. 1a, wobei eine Betätigungsvorrichtung 13a durch eine Kindersicherung 18 blockiert ist.
- Fig. 1c: zeigt eine Detailansicht des Ausführungsbeispiels aus Fig. 1a, wobei die Betätigungsvorrichtung 13a durch die Kindersicherung 18 freigegeben ist.
- Fig. 2: zeigt eine Schnittansicht eines Ausführungsbeispiels der Vorrichtung 1 mit zwei getrennten Zylindern 111, 121, wobei die Kolben 112, 122 mittels einer Stange 162b verbunden sind, die mit einem Hebel 13b2 gekoppelt ist.
- Fig. 3a: zeigt eine Schnittansicht eines Ausführungsbeispiels der Vorrichtung 1 mit zwei getrennten Zylindern 111, 121, wobei eine Betätigungsvorrichtung 13c einstückig mit einer Gewindestange 162c ausgebildet ist.
- Fig. 3b: zeigt eine Schnittansicht eines Ausführungsbeispiels der Vorrichtung 1 mit zwei getrennten Zylindern 111, 121, wobei zwei Kolben 112, 122 einstückig mit einer Gewindestange 162d ausgebildet ist.
- Fig. 4a: zeigt ein Ausführungsbeispiel der Vorrichtung 1 mit einem gemeinsamen Zylinder 16f für beide Tanks 11, 12, wobei ein gemeinsamer Kolben 15f auf einer Gewindestange 13f2 hin und her bewegbar ist.
- Fig. 4b: zeigt einen vergrößerten Ausschnitt aus Fig. 4a, in dem das Eingreifen einer Kanüle 14k in ein Kugelventil 24k eines Verbrauchsmitteltanks 2 dargestellt ist.
- Fig. 5a: zeigt eine Detailansicht eines Ausführungsbeispiels von Vorrichtung 1 und Verbrauchsmitteltank 2, welcher Schnabelventile 24s aufweist.
- Fig. 5b: zeigt eine Detailansicht der Darstellung aus Fig. 5a.
- Fig. 6a: zeigt eine Detailansicht eines Ausführungsbeispiels von Vorrichtung 1 und Verbrauchsmitteltank 2, welcher eine Membran 24m aufweist.
- Fig. 6b: zeigt eine Detailansicht der Darstellung aus Fig. 6a.
- Fig. 7a: zeigt eine Detailansicht des Ausführungsbeispiels aus Fig. 4a, gesehen von rechts.
- Fig. 7b: zeigt eine Detailansicht der Darstellung aus Fig. 7a.
- Fig. 8: zeigt verschiedene Ausführungsbeispiele der Vorrichtung 1, angeschlossen an eine elektrische Zigarette.
- Fig. 9: zeigt ein alternatives Ausführungsbeispiel der Vorrichtung 1 mit einem alternativen Anbindungskonzept zwischen Vorrichtung 1 und Verbrauchsmitteltank 2.
- Fig. 10: zeigt das Ausführungsbeispiel aus Fig. 9 in einer geschnittenen Ansicht.
- Fig. 11: zeigt eine weitere Schnittansicht des Ausführungsbeispiels aus Fig. 9.
- Fig. 12: zeigt eine Detailansicht der Kindersicherung 18 des Ausführungsbeispiels aus Fig. 9.
- Fig. 13: zeigt eine isometrische Detailansicht der Kindersicherung 18 des Ausführungsbeispiels aus Fig. 9.

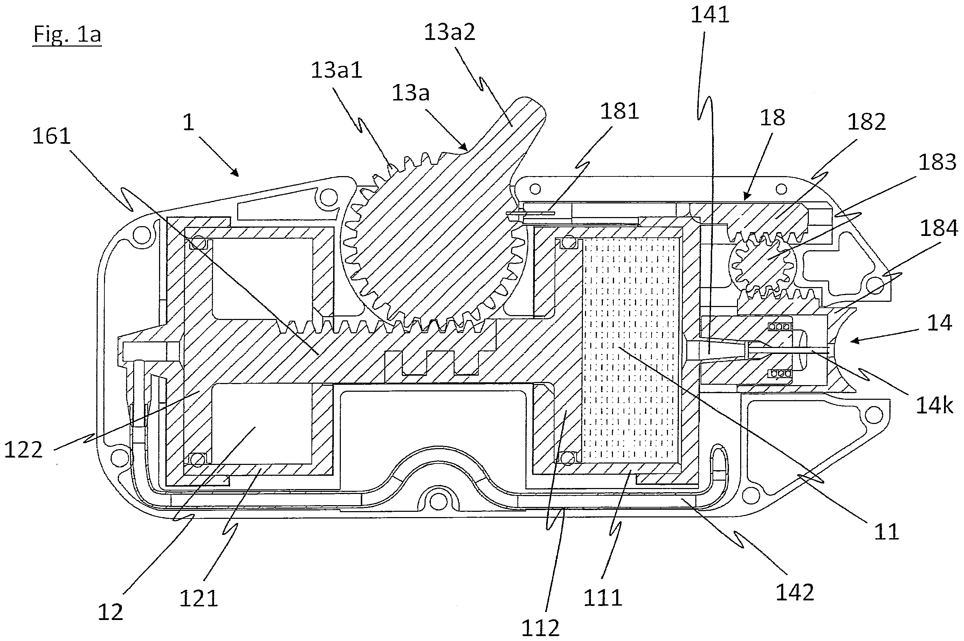

Das in Fig. 1a dargestellte Ausführungsbeispiel der Vorrichtung 1 weist einen ersten Speicherraum in Form eines ersten Tanks 11 zum Aufnehmen eines Verbrauchsmittels und einen zweiten Speicherraum in Form eines zweiten Tanks 12 zum Aufnehmen von Luft oder einem anderen Ausgleichsfluid auf. Der erste Tank 11 weist einen ersten Zylinder 111 als Hohlzylinder und einen ersten Kolben 112 auf. Zum Abdichten zwischen Kolben 112 und Zylinder 111 ist zwischen Zylindermantel und Kolbenumfang eine Ringdichtung angebracht. Der zweite Tank 12 weist einen zweiten Zylinder 121 als Hohlzylinder und einen zweiten Kolben 122 auf, welche analog zum ersten Tank 11 abgedichtet sind. Der erste Tank 11 ist fluidisch mit einem ersten Anschluss 141 verbunden. Der zweite Tank 12 ist fluidisch mit einem zweiten Anschluss 142 verbunden. Die Kolben 112, 122 sind durch eine Zahnstange 161 miteinander verbunden, wobei die Zahnstange 161 im Ausführungsbeispiel zur leichteren Montage zweistückig ausgeführt ist, und jeder der Stücke eine Einheit mit jeweils einem Kolben 112, 122 bildet. In die Zahnstange 161 greift eine Betätigungsvorrichtung 13a mit einem Zahnradabschnitt 13a1 ein. Die Betätigungsvorrichtung 13a weist des Weiteren einen Hebel 13a2 auf.

In der in Fig. 1a gezeigten Darstellung ist der Hebel 13a2 der Betätigungsvorrichtung 13a nach rechts umgelegt. Durch ein Umlegen des Hebels aus der dargestellten Position heraus nach links würde die Einheit aus Gewindestange 161, erstem Kolben 112 und zweitem Kolben 122 bezogen auf die Figur nach rechts bewegt werden. Dabei wird das im ersten Tank 11 enthaltene Verbrauchsmittel, durch den ersten Kanal 141 hindurch, durch die Kanüle 14k gedrückt. Gleichzeitig wird durch den zweiten Kolben 122 Luft aus dem zweiten Anschluss 142 (durch eine weitere, in der Darstellung nicht zu erkennende Kanüle 14k) in den zweiten Tank 12 gesaugt. Der erste Kanal 141 ist, wie in Fig. 1a zu erkennen, fluidisch mit einer Kanüle 14k verbunden. Der zweite Kanal 142 ist fluidisch mit einer zweiten Kanüle 14k verbunden, welche in der gezeigten Darstellung deshalb nicht zu erkennen ist, weil sie hinter der Bildebene liegt.

Des Weiteren ist in Fig. 1a eine Kindersicherung 18 dargestellt, welche einen Riegel 181, einen ersten Schlitten 182, ein Zahnrad 183, sowie einen zweiten Schlitten 184 aufweist. Der zweite Schlitten 184 ist in der in Fig. 1a gezeigten Darstellung so angeordnet, dass sich die Kanülen 14k innerhalb des zweiten Schlittens 184 befinden. Der erste Schlitten 182 und der zweite Schlitten 184 sind horizontal verschiebbar angeordnet, wobei das Zahnrad 183 zwar axial festgelegt, aber drehbar ist. Der Riegel 181 ist fest mit dem ersten Schlitten 182 verbunden. Der erste Schlitten 182 und der zweite Schlitten 184 weisen jeweils einen Zahnabschnitt auf und stehen mit dem Zahnrad 183 in Eingriff. Des Weiteren greift der Riegel 181 in den Zahnradabschnitt 13a1 der Betätigungsvorrichtung 13a ein, und blockiert diese. Wenn der zweite Schlitten 184 durch eine äußere Krafteinwirkung nach links bewegt wird, wird dadurch eine Drehung des Zahnrades 183 in Uhrzeigerrichtung verursacht, durch welche wiederum der erste Schlitten 182 und der Riegel 181 nach rechts bewegt werden. Dabei wird der Riegel 181 aus dem Zahnradabschnitt 13a1 der Betätigungsvorrichtung 13a herausbewegt, und die Kanülen 13k werden durch den zweiten Schlitten 184 nicht mehr abgedeckt, die Kanülenspitzen befinden sich danach vielmehr außerhalb des zweiten Schlittens 184. Der zweite Schlitten 184 fährt dabei hinter die Kanülenspitzen zurück und gibt diese frei.

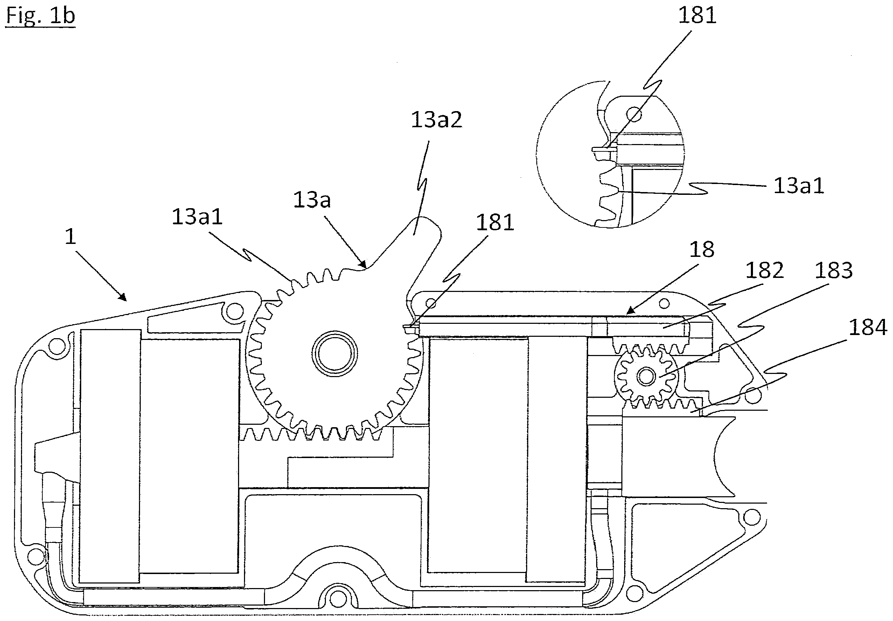

In der in Fig. 1b gezeigten Darstellung befindet sich die Kindersicherung 18, genauer der Riegel 181 in einer verriegelten Position. In dieser verriegelten Position greift der Riegel 181 in den Zahnradabschnitt 13a1 des Betätigungselements 13a ein. Dadurch wird das Betätigungselement 13a blockiert. Ein Bewegen bzw. ein Drehen der Betätigungsvorrichtung 13a durch Krafteinwirkung auf den Hebel 13a2 ist in diesem Zustand nicht möglich. Demnach ist es in diesem Zustand der Kindersicherung 18 nicht möglich, Verbrauchsmittel aus dem ersten Tank 11 auszustoßen.

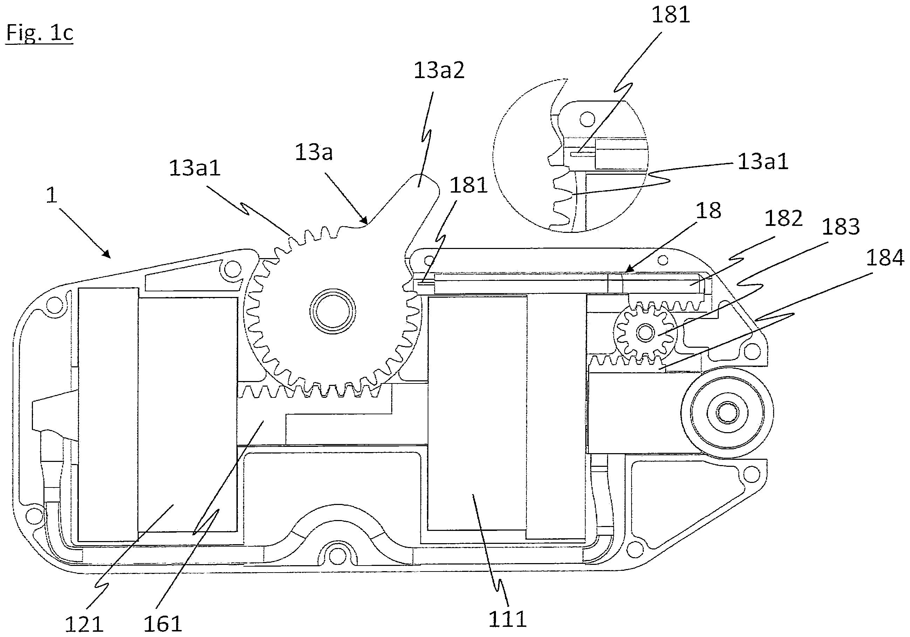

Fig. 1c zeigt das Ausführungsbeispiel aus den Fig. 1a und 1b, wobei sich die Kindersicherung 18 aber in einem nichtverriegelten Zustand, also in einem freigegebenen Zustand befindet. Durch eine äußere Krafteinwirkung auf den zweiten Schlitten 184 wurde dieser nach links verschoben, wodurch das Zahnrad 183 im Uhrzeigersinn gedreht wurde, wodurch der erste Schlitten 182 nach rechts geschoben wurde, und damit den Riegel 181 aus dem Zahnradabschnitt 13a1 der Betätigungsvorrichtung 13a herausbewegt hat. In der in Fig. 1c gezeigten Darstellung ist die Betätigungsvorrichtung 13a demnach nicht durch die Kindersicherung 18 blockiert. Durch eine Krafteinwirkung auf den Hebel 13a2 von rechts nach links ist ein Bewegen der Gewindestange 161 und damit der Kolben 112 und 122 von links nach rechts möglich, wodurch Verbrauchsmittel ausgestoßen und Luft eingesaugt wird, jeweils durch eine der Kanülen 14k.

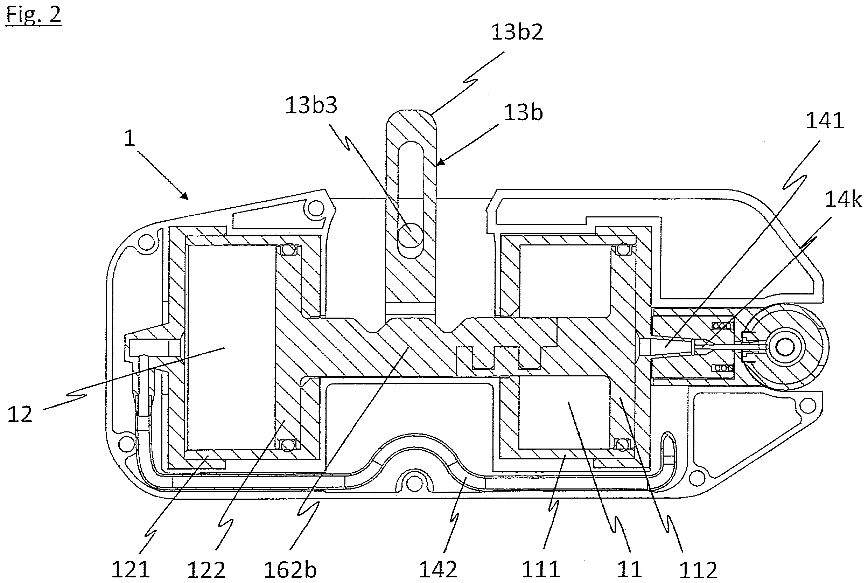

Fig. 2 zeigt ein Ausführungsbeispiel der Vorrichtung 1 ohne Kindersicherung 18, wobei die Betätigungsvorrichtung 13b nicht als Zahnrad wie in den vorherigen Ausführungsbeispielen ausgeführt ist, sondern als Hebel 13b2 mit Drehpunkt 13b3 an des Hebels Angelpunkt. Zwischen einer Stange 162b und der Betätigungsvorrichtung 13b wirkt ein nicht-dargestelltes Gelenk. Der Drehpunkt 13b3 ist fest am Gehäuse der Vorrichtung 1 angeordnet. Durch ein Umlegen des oberen Endes des Hebels 13b2 von links nach rechts resultiert aufgrund des Drehpunkts 13b3 ein Verschieben der Stange 162b von rechts nach links. Die Betätigungsvorrichtung 13b weist zudem ein Langloch auf, in welches der Drehpunkt 13b3 eingreift. Das Langloch erlaubt, dass sich der Abstand vom oben erwähnten nicht-dargestellten Gelenk zum Drehpunkt 13b3 bei einem Umlegen des Hebels 13b2 verändert. Die restlichen Bauteile sind analog zu dem gezeigten Ausführungsbeispiel aus Fig. 1a, 1b und 1c zu verstehen.

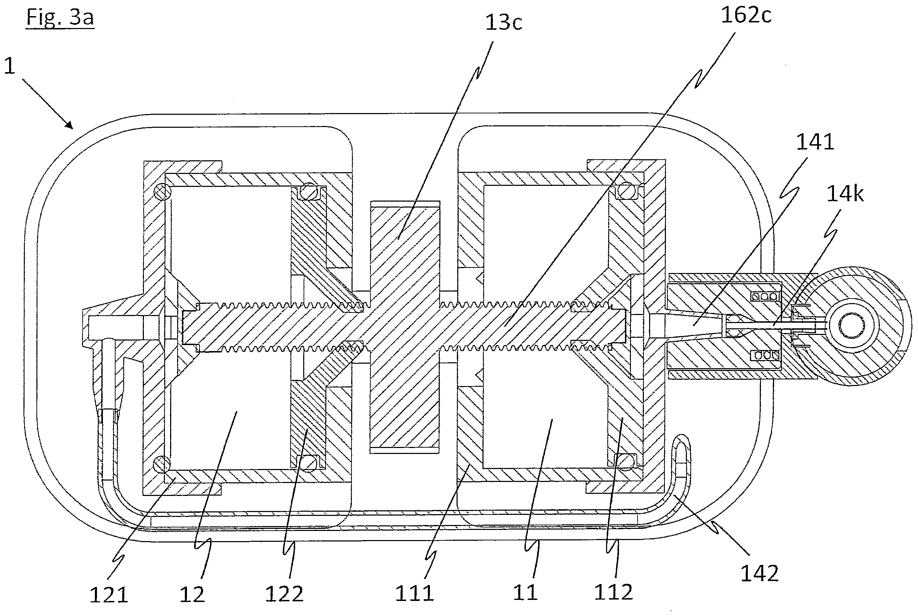

Das in Fig. 3a gezeigte Ausführungsbeispiel weist einen ersten Tank 11 zum Aufnehmen eines Verbrauchsmittels und einen zweiten Tank 12 zum Aufnehmen von Luft oder einem anderen Ausgleichsfluid auf. Der erste Tank 11 weist einen ersten Zylinder 111 und einen ersten Kolben 112 auf. Der zweite Tank 12 weist einen zweiten Zylinder 121 und einen zweiten Kolben 122 auf, analog zu den bisher vorgestellten Ausführungsbeispielen. Die Betätigungsvorrichtung 13c weist allerdings ein Stellrad auf. Die Betätigungsvorrichtung 13c ist einstückig mit einer Gewindestange 162c verbunden.

In einer Abwandlung dieses Ausführungsbeispiels ist die Betätigungsvorrichtung 13c zwar drehfest mit der Gewindestange 162c verbunden, aber nicht einstückig mit dieser ausgeführt.

Durch Drehen der Betätigungsvorrichtung 13c um eine horizontal in der Bildebene verlaufenden Achse wird die Gewindestange 162c aufgrund der Einstückigkeit oder drehfesten Verbindung zwischen Betätigungsvorrichtung 13c und Gewindestange 162c mitgedreht. Der erste Kolben 112 sowie der zweite Kolben 122 weisen jeweils ein Innengewinde auf. Diese Innengewinde stehen in Gewindeeingriff mit der Gewindestange 162c. Wenn die Gewindestange 162c mittels der Betätigungsvorrichtung 13c gedreht wird, verschieben sich die Kolben 112, 122 auf der Gewindestange nach links und rechts, je nach Drehrichtung. Dabei werden die Kolben 112, 122 an einem Mitdrehen mit der Gewindestange 162c auf Grund der Reibung zwischen Ringdichtung, Kolben 112, 122 und Zylinder 111, 121 gehindert. In einem nicht dargestellten Ausführungsbeispiel werden die Kolben 112, 122 in den Zylindern 111, 121 mittels Feder und Nut geführt, um ein Mitdrehen der Kolben 112, 122 mit der Gewindestange 162c zu verhindern. Alle restlichen Elemente sind analog zu den bisher vorgestellten Ausführungsbeispielen zu verstehen.

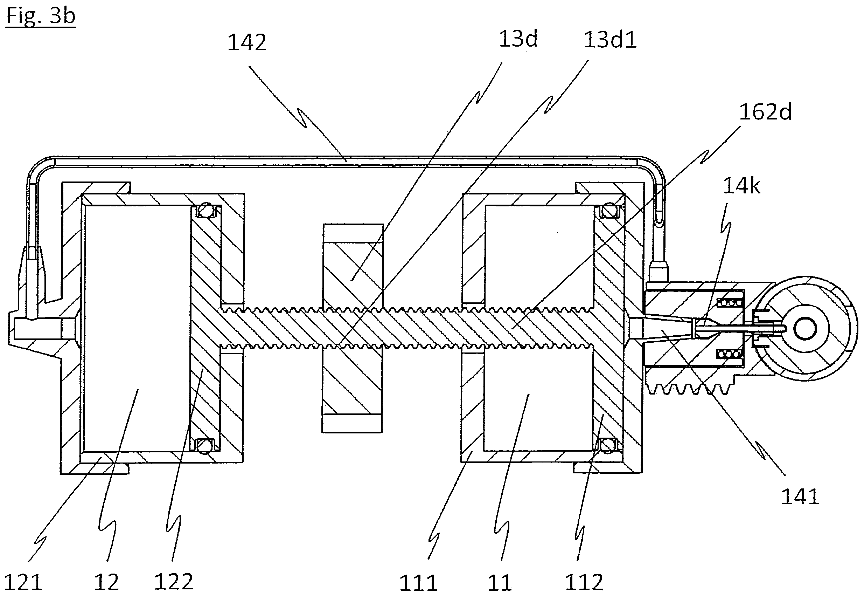

Das in Fig. 3b gezeigte Ausführungsbeispiel zeigt eine Abwandlung des in Fig. 3a gezeigten Ausführungsbeispiels, allerdings ist in dem in Fig. 3b gezeigten Ausführungsbeispiel die Gewindestange 162d nicht mit der Betätigungsvorrichtung 13d fest verbunden oder einstückig mit dieser ausgebildet. Vielmehr sind der erste Kolben 112 und der zweite Kolben 122 fest mit der Gewindestange 162d verbunden. Die Betätigungsvorrichtung 13d weist ein Innengewinde 13d1 auf, welches in Gewindeeingriff mit der Gewindestange 162d steht. Die Betätigungsvorrichtung 13d wird des Weiteren durch nicht dargestellte Flanken des Gehäuses der Vorrichtung 1 axial gehalten, um die Betätigungsvorrichtung 13d an einem Bewegen nach links und rechts zu hindern. Durch Drehen des Betätigungselements 13d ist die Gewindestange 162d zusammen mit den Kolben 112, 122 somit in der Bildebene nach links und rechts verschiebbar. Alle restlichen Elemente sind analog zu den bisher vorgestellten Ausführungsbeispielen zu verstehen.

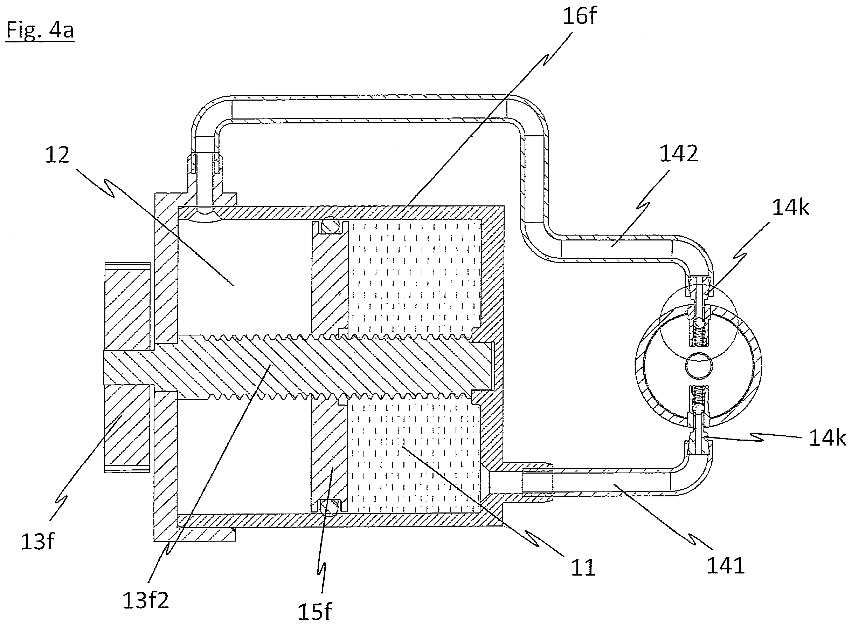

Das in Fig. 4a dargestellte Ausführungsbeispiel der Vorrichtung 1 weist einen gemeinsamen Zylinder 16f für den ersten Tank 11 und den zweiten Tank 12 auf. Die beiden Tanks 11, 12 werden durch einen gemeinsamen Kolben 15f voneinander getrennt. Der Kolben 15f weist ein Innengewinde auf, welches in Gewindeeingriff mit einer Gewindestange 13f2 steht. Mit der Gewindestange 13f2 ist eine Betätigungsvorrichtung 13f fest verbunden. In einer Abwandlung des Ausführungsbeispiels sind Gewindestange 13f2 und Betätigungsvorrichtung 13f einstückig ausgebildet.

Wird die Betätigungsvorrichtung 13f zusammen mit der Gewindestange 13f2 um eine horizontal in der Bildebene verlaufende Achse gedreht, wird der mit der Gewindestange 13f2 in Gewindeeingriff stehende Kolben 15f nach links oder rechts geschoben. Bei einer Bewegung des Kolbens 15f nach rechts wird Verbrauchsmittel aus dem Tank 11 durch den ersten Anschluss 141 hindurch gefördert, wobei Luft aus dem zweiten Anschluss 142 in den zweiten Tank 12 gesaugt wird. Analog zu den vorherigen Ausführungsbeispielen wird ein Mitdrehen des Kolbens 15f, gemeinsam mit der Gewindestange 13f2 beim Drehen an der Betätigungsvorrichtung 13f, verhindert.

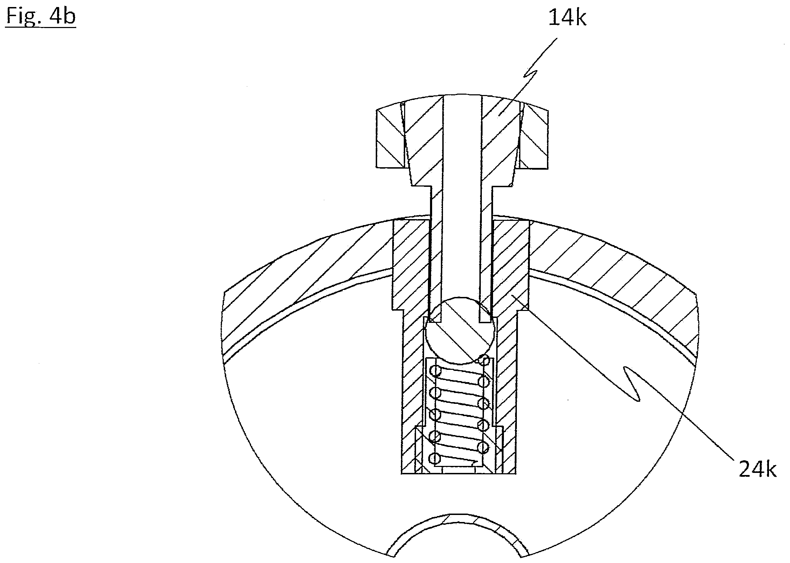

Fig. 4b zeigt das Eingreifen einer Kanüle 14k der Vorrichtung 1 in ein Kugelventil 24k eines Verbrauchsmitteltanks 2.

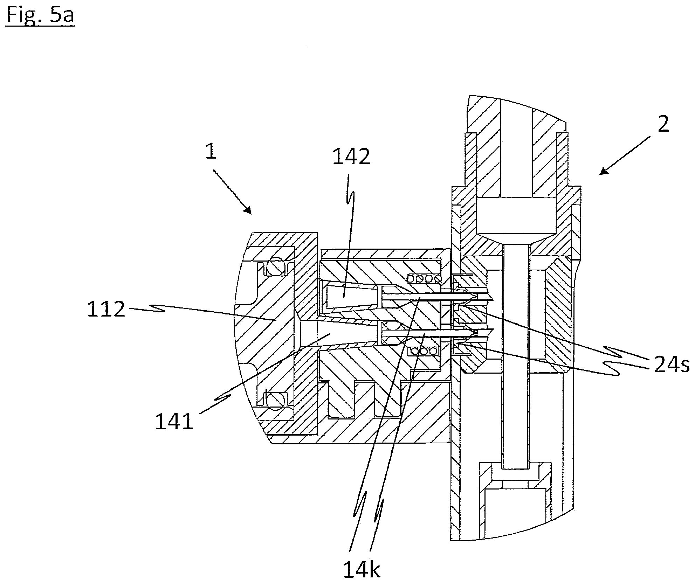

Fig. 5a zeigt das Eingreifen von zwei Kanülen 14k der Vorrichtung 1 in zwei Schnabelventile 24s des Verbrauchsmitteltanks 2. Die untere Kanüle 14k ist fluidisch mit dem ersten Anschluss 141 verbunden, welcher wiederum fluidisch mit dem ersten Tank 11 verbunden ist. Die obere Kanüle 14k ist fluidisch mit dem zweiten Anschluss 142 verbunden, welcher wiederum fluidisch mit dem zweiten Tank 12 verbunden ist. Die Darstellung in Fig. 5a zeigt das Eingreifen der Vorrichtung 1 in einen Verbrauchsmitteltank 2 einer elektrischen Zigarette, nachdem der Befüllvorgang der elektrischen Zigarette abgeschlossen ist. Der Kolben 112 des ersten Tanks 11 befindet sich in einer komplett nach rechts bewegten Position, in der der gesamte Inhalt des ersten Tanks 11 bereits durch den ersten Kanal 141 und durch die untere Kanüle 14k hindurch in den Verbrauchsmitteltank 2 der elektrischen Zigarette gefördert wurde. Der Verbrauchsmitteltank 2 wird mit einem oberen und einem unteren Schnabelventil 14s abgedichtet. Die Anordnung der Kanülen 14k und der Schnabelventile 24s in der Konfiguration "Einfüllen unten, Absaugen oben" dient dazu, dass in den Verbrauchsmitteltank 2 in einem unteren Bereich Verbrauchsmittel zugeführt wird, und ein verdrängtes Luftvolumen in einem oberen Bereich abgesaugt wird. Aufgrund des Dichteunterschieds von Verbrauchsmittel und Luft würde eine andere Anordnung keinen Sinn machen, da sonst das eingefüllte Verbrauchsmittel direkt wieder abgesaugt werden würde.

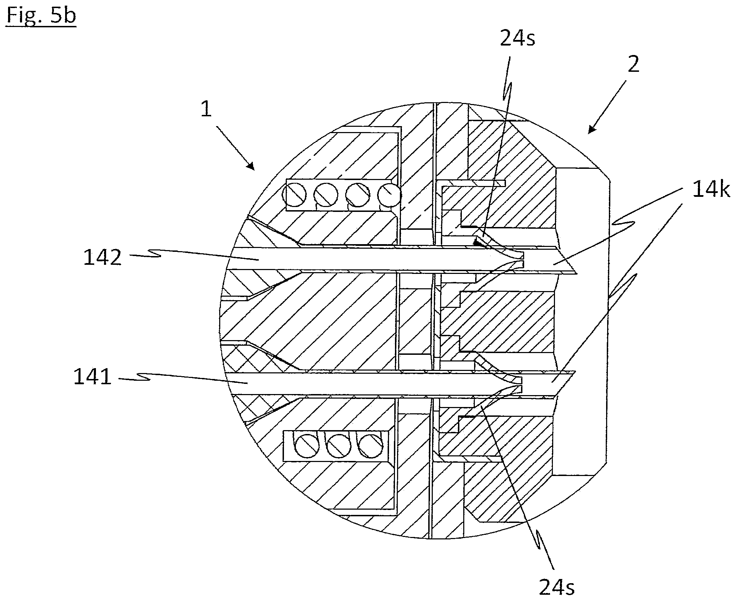

Fig. 5b zeigt Detailansicht der Darstellung aus Fig. 5a. In dieser Darstellung sind die Schnäbel der Schnabelventile 24s überlappend mit den Kanülen 14k schematisch dargestellt. Die Schnäbel liegen nach dem Eindringen der Kanülen an den äußeren Mänteln der Kanülen 14k an. Die schematische Darstellung der Schnäbel zeigt einen geschlossenen Zustand.

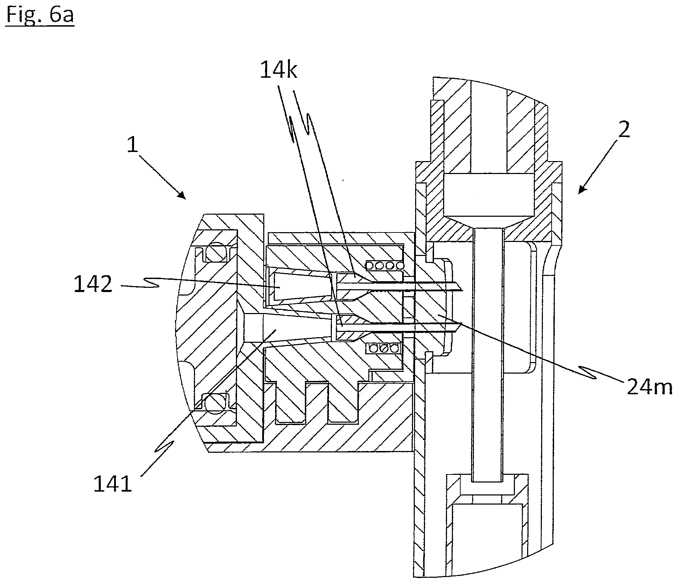

Fig. 6a zeigt das Ausführungsbeispiel aus Fig. 5a, wobei anstatt der Schnabelventile 24s eine Membran 24m zum Abdichten des Verbrauchsmittelstanks 2 vorgesehen ist. Diese Membran 24m kann als gummiartiges Bauteil vorgesehen sei, welches mittels der Kanülen 14k durchstochen wird. Durch ein Herausziehen der Kanülen 14k nach dem Befüllen des Verbrauchsmitteltanks 2 ziehen sich die in die Membran 24m gestochenen Öffnungen wieder zusammen und dichten den Verbrauchsmitteltank 2 ab. Die Membran 24m ist als mehrfach verwendbares Wegwerfprodukt vorgesehen, welches austauschbar ist, wenn die Abdichtwirkung nicht mehr gegeben ist.

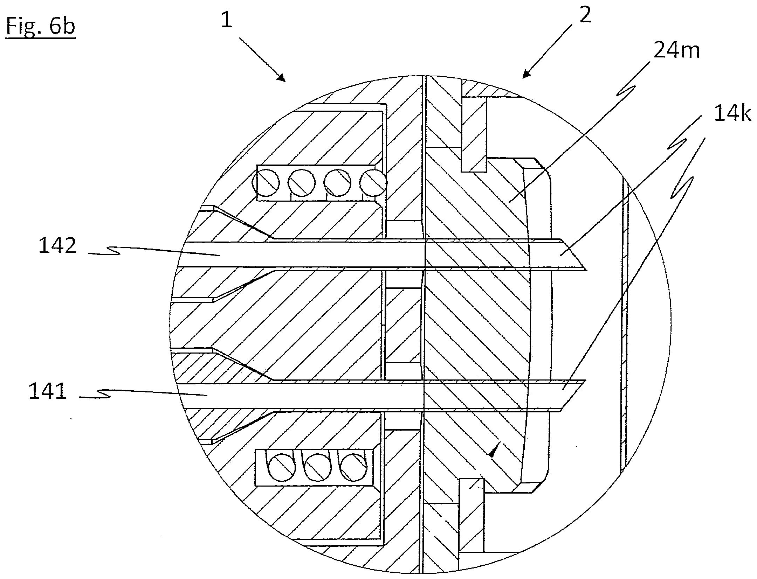

Fig. 6b zeigt eine Detailansicht der Darstellung aus Fig. 6a. In dieser ist innerhalb der Kanülen 14k die Schraffur der Membran 24m zu erkennen. Diese Darstellung entspricht einem Zustand, in welchem die Membran 24m unmittelbar mittels der Kanülen 14k durchstocken wurde. Sobald ein Fluidaustausch (Befüll- und Absaugvorgang) stattfindet, bewegen sich die ausgestochenen Membranstücke aus den Kanülen 14k heraus.

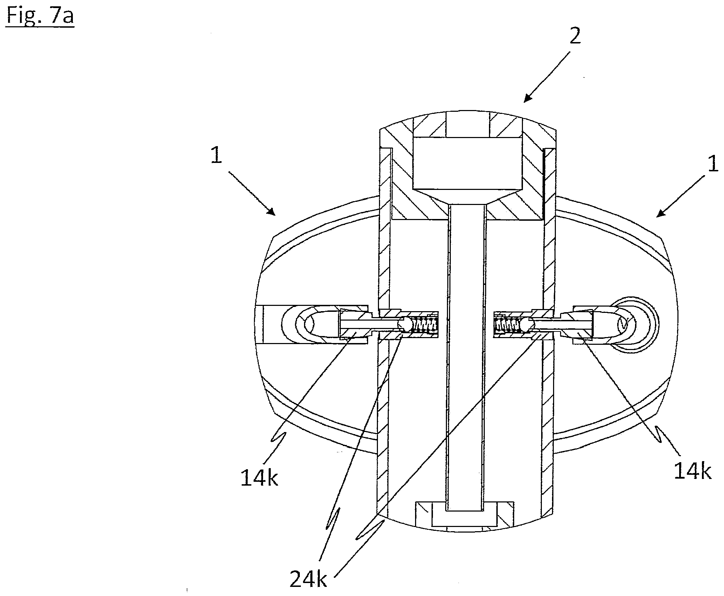

Fig. 7a zeigt das in Fig. 4a dargestellte Ausführungsbeispiel der Vorrichtung 1, welches sich in einem an den Verbrauchsmitteltank 2 angeschlossenen Zustand befindet. In der gezeigten Darstellung wird der Verbrauchsmitteltank 2 von verschiedenen Seiten, von links und rechts, kontaktiert. Es sind auf der linken und auf der rechten Seite jeweils eine Kanüle 14k der Vorrichtung 1 und jeweils ein Kugelventil 24k des Verbrauchsmitteltanks 2 dargestellt.

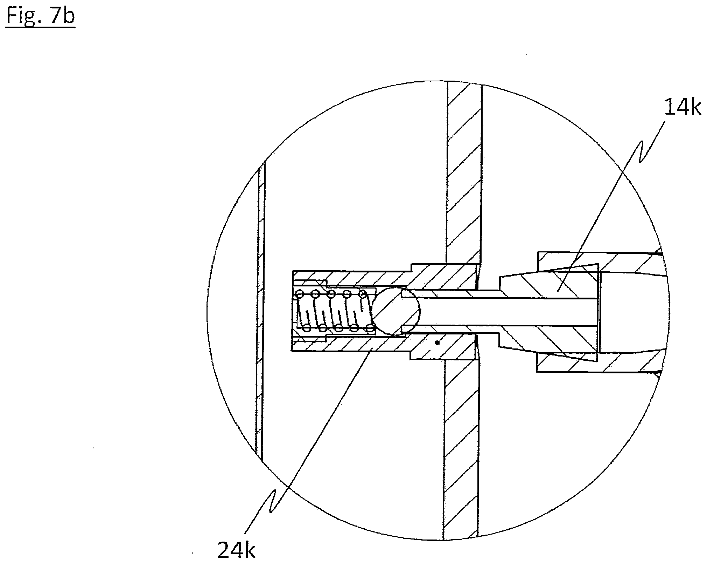

Fig. 7b zeigt eine Detailansicht der Darstellung aus Fig. 7a. Es ist das Zusammenwirken von Kanüle 14k der Vorrichtung 1 und Kugelventil 24k des Verbrauchsmitteltanks 2 zu erkennen.

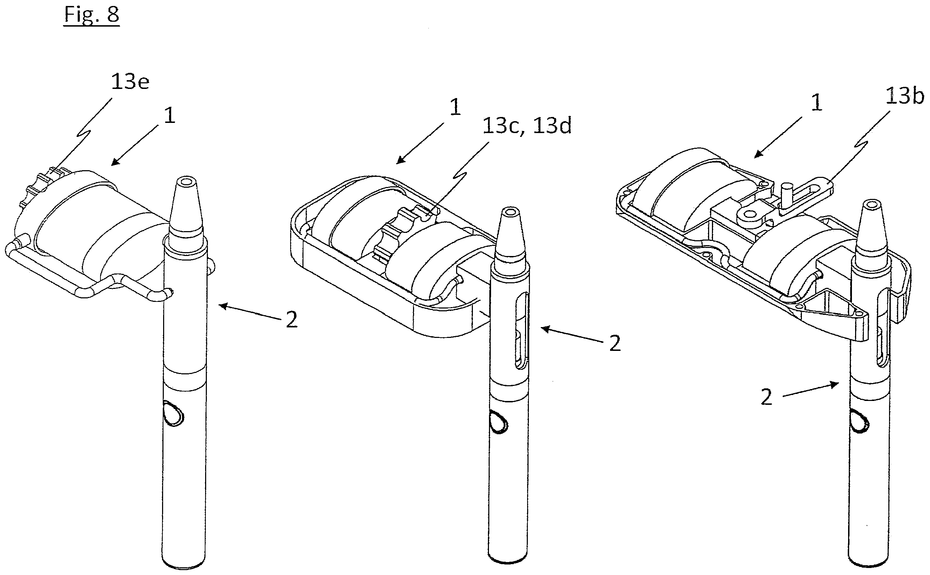

Fig. 8 zeigt isometrische Ansichten von verschiedenen Ausführungsformen der Vorrichtung 1, während diese an einen Verbrauchsmitteltank 2 einer elektrischen Zigarette angeschlossen sind. Ganz links wird das Ausführungsbeispiel aus Fig. 4a dargestellt, mittig werden die Ausführungsbeispiele aus Fig. 3a und Fig. 3b dargestellt, wobei auf der rechten Seite das Ausführungsbeispiel aus Fig. 2 dargestellt ist.

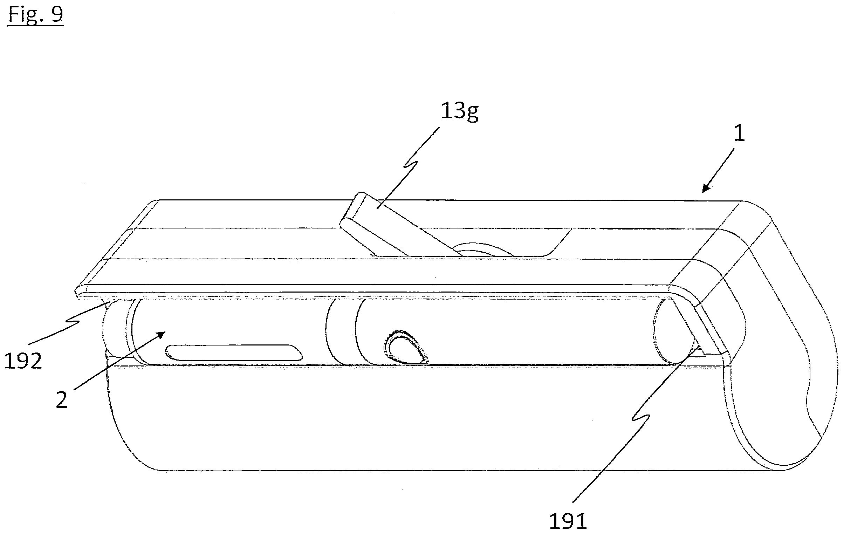

Fig. 9 zeigt eine isometrische Ansicht eines alternativen Anbindungskonzepts zwischen Vorrichtung 1 und elektrischer Zigarette bzw. Verbrauchsmitteltank 2. Bei den vorherig erläuterten Ausführungsbeispielen (siehe insbesondere Fig. 8) werden die Vorrichtung 1 und die zu befüllende elektrische Zigarette in einer Position miteinander gekoppelt, in welcher die Hauptkörperachsen von Vorrichtung 1 und elektrischer Zigarette einen Winkel von ungefähr 90 Grad aufweisen. Um den Kopplungsvorgang zwischen Vorrichtung 1 und elektrischer Zigarette bzw. Verbrauchsmitteltank 2 zu vereinfachen, weist die Vorrichtung 1 dieses Ausführungsbeispiels ein Schalenelement mit einer Aufnahme 192 zum Einlegen der elektrischen Zigarette auf. Die Innenkontur der Aufnahme 192 korrespondiert mit der Außenkontur der elektrischen Zigarette, sodass diese formschlüssig in die Vorrichtung 1 einlegbar ist. Des Weiteren weist das Schalenelement einen Endanschlag 191 zum Ausrichten der elektrischen Zigarette in ihrer axialen Richtung innerhalb der Vorrichtung 1 auf. Die Vorrichtung 1 weist eine Betätigungsvorrichtung 13g auf, und die Funktionsweise verhält sich im Wesentlichen analog zu der Funktionsweise der vorhergehenden Ausführungsbeispiele.

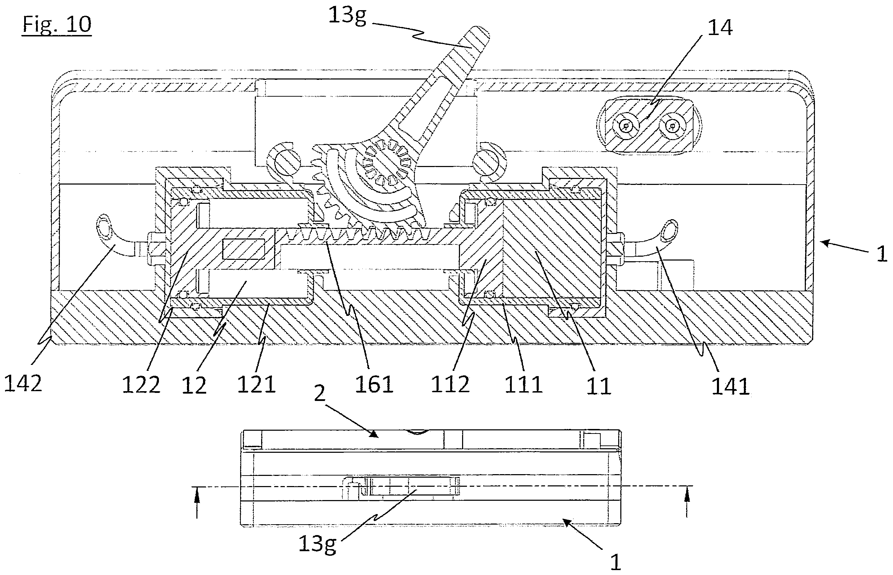

Fig. 10 zeigt im unteren Abschnitt eine Draufsicht auf die Darstellung aus Fig. 9 mit Blick auf die Fläche der Vorrichtung 1, in welcher die Betätigungsvorrichtung 13g angeordnet ist. In dieser Draufsicht ist eine Schnittlinie mit Pfeilen eingezeichnet, welche die Klappungsrichtung des Schnitts zeigen. Der Schnitt ist vergrößert im oberen Abschnitt von Figur 10 dargestellt. Analog zu der Darstellung in Fig. 1a werden der erste Tank 11 und der zweite Tank 12 gezeigt, wobei jeder der Tanks 11, 12 einen Zylinder 111, 121 sowie einen Kolben 112, 122 aufweist. Des Weiteren werden die Anschlüsse 141 und 142 dargestellt, welche außerhalb der Schnittebene zu der Schnittstelle 14 verlaufen. Des Weiteren sind die Kolben 112, 122 mittels einer Zahnstange 161 miteinander gekoppelt.

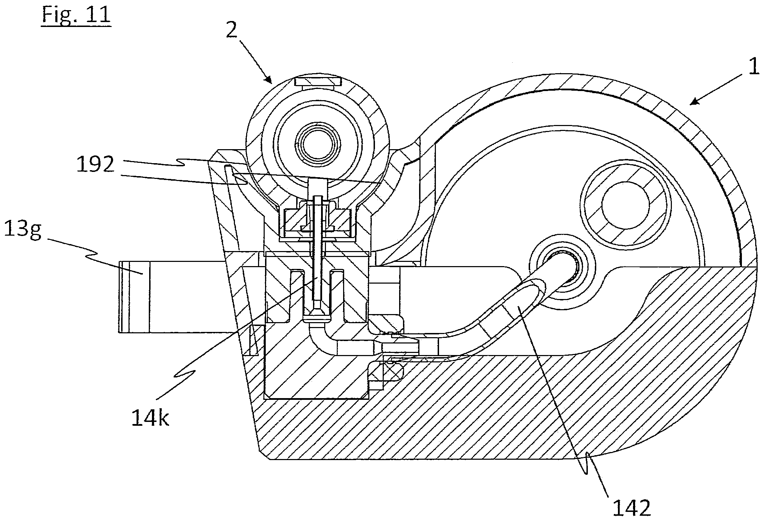

Fig. 11 zeigt eine weitere Schnittansicht des Ausführungsbeispiels aus Fig. 9, senkrecht durch die Hauptachse der elektrischen Zigarette geschnitten. Es sind die elektrische Zigarette bzw. der Verbrauchsmitteltank 2 dargestellt, in einer mit der Vorrichtung 1 gekoppelten Position. Die elektrische Zigarette befindet sich dabei innerhalb der Aufnahme 192 bzw. innerhalb des Schalenelements.

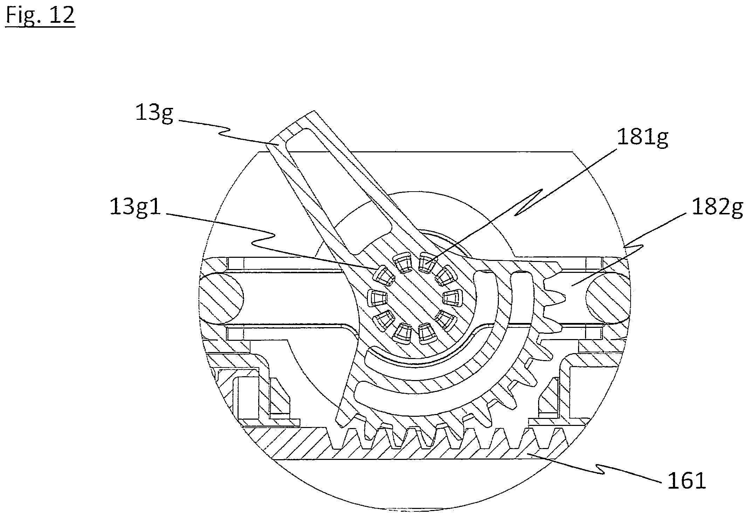

Fig. 12 zeigt eine geschnittene Detailansicht des Ausführungsbeispiels aus Fig. 9, anhand welcher die Funktionsweise der Kindersicherung 18 erläutert wird. Die Kindersicherung 18 weist einen Riegel 182g auf, der kreisförmig auf dem Riegel 182g angeordnete Zapfen 181g aufweist. Des Weiteren weist die Betätigungsvorrichtung 13g kreisförmig angeordnete Ausnehmungen 13g1 auf, welche so angeordnet sind, dass ein Eingreifen der Zapfen 181g in die Ausnehmungen 13g1 möglich ist. Wenn die Zapfen 181g in die Ausnehmungen 13g1 eingreifen, wird dadurch die Betätigungsvorrichtung 13g blockiert. Dieser blockierte Zustand wird mittels Federelemente dann aufrechterhalten, wenn sich an der Vorrichtung 1 keine elektrische Zigarette in einer gekoppelten Position befindet. Hierzu ist ein die Aufnahme für die elektrische Zigarette ausbildender Teil der Vorrichtung 1 so mit der Kindersicherung 18 gekoppelt, dass die Zapfen 181g durch ein Einlegen einer elektrischen Zigarette (in die Vorrichtung 1) in die Zeichenebene hinein (vom Betrachter weg) verschoben werden, und die Betätigungsvorrichtung 13g somit freigegeben wird. Daraufhin kann die Betätigungsvorrichtung 13g zum Befüllen des Verbrauchsmitteltanks 2 bewegt werden. Durch ein Herausnehmen der elektrischen Zigarette aus der Vorrichtung 1 nach dem Befüllvorgang des Verbrauchsmitteltanks 2 werden die Zapfen 181g mittels Federkraft wieder in Richtung des Betrachters (von Fig. 12) zurückbewegt, sodass die Zapfen 181g wieder in die Ausnehmungen 13g1 eingreifen, und die Betätigungsvorrichtung 13g somit blockiert wird.

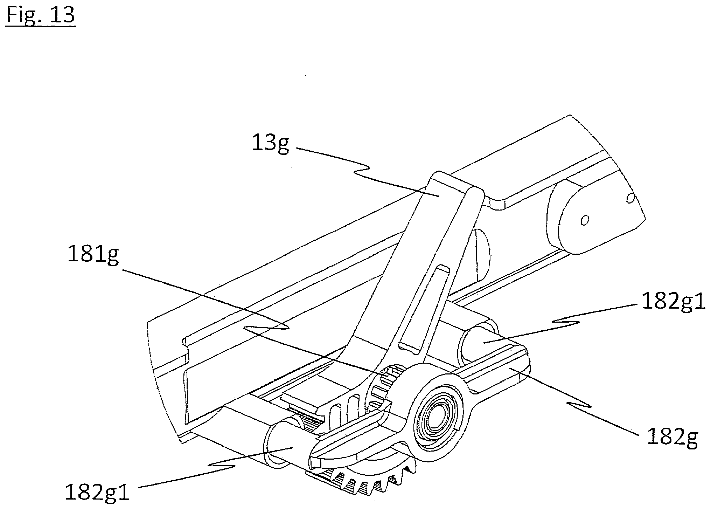

Fig. 13 zeigt die Kindersicherung 18 in einer perspektivischen Ansicht, um deren Funktionsweise zu verdeutlichen. Der Riegel 182g weist zwei Kolben 182g1 auf, welche ein Führen des Riegels 182g im Gehäuse der Vorrichtung 1 ermöglichen. Des Weiteren ist der die Aufnahme für die elektrische Zigarette ausbildende Teil der Vorrichtung 1 so mit den Kolben 182g1 gekoppelt, dass der Riegel 182g bzw. die Zapfen 181g aus der Betätigungsvorrichtung 13g herausbewegt werden, wenn eine elektrische Zigarette in die Vorrichtung 1 eingelegt wird. Dadurch wird die Betätigungsvorrichtung 13g freigegeben, wie in Fig. 13 dargestellt. Durch eine in dieser Ansicht nicht dargestellte Feder wird der Riegel 182g in eine die Betätigungsvorrichtung 13g blockierende Position (zurück) gedrückt, in welcher die Zapfen 181g in die Betätigungsvorrichtung 13g eingreifen.

Wie bereits erläutert, sind dem Fachmann Abwandlungen der oben beschriebenen Ausführungsbeispiele offensichtlich, die den durch die Ansprüche definierten Schutzbereich nicht verlassen. So sind insbesondere aus den Ausführungsbeispielen aus Fig. 1a, 2 und 10 die Kolben 112, 122 derart mit der Zahnstange 161 gekoppelt, dass eine einstückige, feste Verbindung vorliegt. Diese Koppelung muss jedoch weder einstückig noch fest ausgestaltet sein. Als Kopplung im Sinne dieser Lehre ist jede feste oder lose, mittelbare oder unmittelbare Wirkverbindung zu verstehen, die erlaubt, Kräfte in einer Form zu übertragen, sodass die beabsichtigte Aufgabe der Gegenstände der einzelnen Ansprüche erfüllt werden kann.

## Patentansprüche

1. Vorrichtung (1) zum Befüllen einer elektrischen
Zigarette, aufweisend
einen ersten Speicherraum (11) zum Aufnehmen eines Verbrauchsstoffs,
eine Schnittstelle (14) mit einem ersten Anschluss (141), der fluidisch mit dem ersten Speicherraum (11) verbunden ist,
wobei der erste Anschluss so konstruiert ist, dass er am entsprechenden ersten Anschluss des Verbrauchsmitteltanks einer elektronischen Zigarette angeschlossen werden kann, **gekennzeichnet durch**,
einen zweiten Speicherraum (12) zum Aufnehmen eines Ausgleichsfluids, wobei
die Vorrichtung (1) so konstruiert ist, dass die Volumina des ersten Speicherraums (11) und des zweiten Speicherraums (12) mittels einer Betätigungsvorrichtung (13a, 13b, 13c, 13d, 13e, 13f, 13g) gegenläufig veränderbar sind, und wobei die Schnittstelle (14)
einen zweiten Anschluss (142) aufweist, der fluidisch mit dem zweiten Speicherraum (12) verbunden ist, wobei auch der zweite Anschluss so konstruiert ist, dass er am entsprechenden zweiten Anschluss des Verbrauchsmitteltanks einer elektronischen Zigarette angeschlossen werden kann.

2. Vorrichtung (1) gemäß dem vorhergehenden Anspruch, wobei der erste Speicherraum (11) einen ersten Hohlzylinder (111) und der zweite Speicherraum (12) einen zweiten Hohlzylinder (121) aufweist, und
eine Volumenänderung ΔV₁ des ersten Speicherraums (11) mittels eines in den ersten Hohlzylinder (111) ein- und ausfahrbaren ersten Kolbens (112) und eine Volumenänderung ΔV₂ des zweiten Speicherraums (12) mittels eines in den zweiten Hohlzylinder (121) ein- und ausfahrbaren zweiten Kolbens (122) herbeiführbar ist.

3. Vorrichtung (1) gemäß einem der vorhergehenden Ansprüche, wobei
die Betätigungsvorrichtung (13a, 13g) einen Zahnradabschnitt (13a1) aufweist,
der erste Kolben (112) und der zweite Kolben (122) mittels einer Betätigungsstange (161) in Form einer Zahnstange (161), Gewindestange oder anderen Stange mit zahnartigen Eingriffselementen mittelbar oder unmittelbar miteinander gekoppelt sind, und
die Vorrichtung (1) so konstruiert ist, dass der Zahnradabschnitt (13a1) und die Betätigungsstange (161) miteinander in Eingriff stehen.

4. Vorrichtung (1) gemäß einem der Ansprüche 1 oder 2, wobei
die Betätigungsvorrichtung (13b) einen Hebel (13b2) aufweist, wobei
der erste Kolben (112) und der zweite Kolben (122) mittelbar oder unmittelbar mit dem Hebel (13b2) gekoppelt sind.

5. Vorrichtung (1) gemäß dem vorhergehenden Anspruch, wobei
die Betätigungsvorrichtung (13b) einen Drehpunkt (13b3) für den Hebel (13b2) aufweist, wobei
der erste Kolben (112) und der zweite Kolben (122) mittels einer Stange (162b) mittelbar oder unmittelbar miteinander gekoppelt sind, wobei
der Hebel (13b2) und die Stange (162b) miteinander gekoppelt sind.

6. Vorrichtung (1) gemäß einem der Ansprüche 1 oder 2,
wobei
die Betätigungsvorrichtung (13c) drehfest mit einer Gewindestange (162c) verbunden ist, wobei
die Kolben (112, 122) ein Innengewinde aufweisen, und
die Vorrichtung (1) so konstruiert ist, dass die Kolben (112, 122) auf der Gewindestange (162c) bezüglich des ersten Hohlzylinders (111) und des zweiten Hohlzylinders (121) verschiebbar sind.

7. Vorrichtung (1) gemäß einem der Ansprüche 1 oder 2,
wobei
die Betätigungsvorrichtung (13d) ein Innengewinde aufweist, wobei
der erste Kolben (112) und der zweite Kolben (122) fest mit einer Gewindestange (162d) verbunden sind, wobei
die Vorrichtung (1) so konstruiert ist, dass die Gewindestange (162d) mit den Kolben (112, 122) bezüglich der Betätigungsvorrichtung (13d) und der Hohlzylinder (111, 121) mittels der Betätigungsvorrichtung (13d) verschiebbar ist.

8. Vorrichtung (1) gemäß einem der Ansprüche 1 oder 2,
wobei
eine Stange (162e) drehfest bezüglich einer Betätigungsvorrichtung (13e), aber axial verschieblich innerhalb der Betätigungsvorrichtung (13e) angeordnet ist, wobei
die Kolben (112, 122) fest mit der Stange (162e) verbunden sind und wenigstens einer der Hohlzylinder (111, 121) ein Innengewinde aufweist, wobei
wenigstens einer der Kolben (112, 122) in das Innengewinde des wenigstens einen Hohlzylinders (111, 121) mittels einem Außengewinde oder wenigstens einem Zapfen eingreift, wobei
die Vorrichtung (1) so konstruiert ist, dass die Stange (162e) mit den Kolben (112, 122) bezüglich der Zylinder (111, 121) und der Betätigungsvorrichtung (13e) mittels der Betätigungsvorrichtung (13e) verschiebbar ist.

9. Vorrichtung (1) gemäß Anspruch 1, wobei
der erste und der zweite Speicherraum (11, 12) einen gemeinsamen Hohlzylinder (16f) aufweisen und Volumenänderungen ΔV₁, ΔV₂ mittels einem in dem gemeinsamen Hohlzylinder (16f) bewegbaren Kolben (15f) herbeiführbar ist.

10. Vorrichtung (1) gemäß dem vorhergehenden Anspruch, wobei
die Betätigungsvorrichtung (13f) eine Gewindestange (13f2) mit Außengewinde und der Kolben (15f) ein Innengewinde aufweist.

11. Vorrichtung (1) gemäß einem der vorhergehenden Ansprüche, wobei
die Vorrichtung (1) so konstruiert ist, dass in den Speicherräumen (11, 12) entweder Volumenänderungen ΔV₁, ΔV₂ von gleicher oder von ungleicher Größe herbeiführbar sind.

12. Vorrichtung (1) gemäß einem der vorhergehenden Ansprüche, wobei
der ersten Anschluss (141) eine erste Kanüle (14k) und der zweite Anschluss (142) eine zweite Kanüle (14k) aufweist.

13. Vorrichtung (1) gemäß einem der vorhergehenden Ansprüche, des Weiteren aufweisend eine Kindersicherung (18), die zum Blockieren und Freigeben der Betätigungsvorrichtung (13a, 13b, 13c, 13d, 13e, 13f, 13g) mit einem Riegel (181, 182g) versehen ist.

14. Vorrichtung (1) gemäß dem vorhergehenden Anspruch, wobei die Kindersicherung (18) so konstruiert ist, dass diese die Betätigungsvorrichtung (13a, 13g) freigibt, wenn sich die Vorrichtung (1) in einem Freigabezustand befindet, und die Betätigungsvorrichtung (13a, 13g) andernfalls blockiert.

15. Vorrichtung (1) gemäß einem der vorhergehenden Ansprüche, aufweisend ein Schalenelement, das zum Aufnehmen einer elektrischen Zigarette vorgesehen und dazu eingerichtet ist, die Lage und Ausrichtung einer elektrischen Zigarette in einem, zwei, drei, vier, fünf oder sechs Freiheitsgraden zu definieren.

16. Verbrauchsmitteltank (2) einer elektrischen Zigarette, aufweisend
einen ersten Anschluss zum befüllt werden mit Verbrauchsmittel,
**dadurch gekennzeichnet, dass**
der Verbrauchsmitteltank (2) ferner einen zweiten Anschluss zum Ablassen eines Ausgleichsfluids, insbesondere von Luft aufweist, wobei der erste Anschluss und der zweite Anschluss so konstruiert sind, dass sie jeweils am entsprechenden ersten und zweiten Anschluss von der Vorrichtung gemäß Anspruch 1 angeschlossen werden können.

17. Verbrauchsmitteltank (2) gemäß dem vorhergehenden Anspruch, wobei jeder der Anschlüsse (241, 242) wenigstens
eine Membran (24m) oder
ein Schnabelventil (24s) oder
ein Kugelventil (24k) aufweist.

18. System (3) aus einer Vorrichtung (1) gemäß einem der vorhergehenden Ansprüche 1 bis 15 und einem Verbrauchsmitteltank (2) gemäß einem der Ansprüche 16 bis 17.

19. System (3) aus einer Vorrichtung (1) gemäß einem der vorhergehenden Ansprüche 13 bis 15 und einem Verbrauchsmitteltank (2) gemäß einem der Ansprüche 16 bis 17, wobei
die Kindersicherung (18) so konstruiert ist, dass diese die Betätigungsvorrichtung (13a, 13g) freigibt, wenn sich die Vorrichtung (1) und der Verbrauchsmitteltank (2) bezüglich einender in einer Befüllposition befinden, und die Kindersicherung (18) die Betätigungsvorrichtung (13a, 13g) andernfalls blockiert.

20. Verfahren zum Befüllen eines Verbrauchsmitteltanks (2) einer elektrischen Zigarette mittels einer Vorrichtung (1) zum Befüllen einer elektrischen Zigarette, aufweisend den Schritt:
- Betätigen einer Betätigungsvorrichtung (13a, 13b, 13c, 13d, 13e, 13f, 13g), wodurch Verbrauchsmittel aus der Vorrichtung (1) in den Tank (2) einer elektrischen Zigarette eingefüllt wird,
**dadurch gekennzeichnet, dass**
gleichzeitig ein durch das eingefüllte Verbrauchsmittel verdrängtes Ausgleichsfluidvolumen aus dem Tank (2) der Zigarette abgesaugt wird.

21. Verfahren gemäß dem vorhergehenden Anspruch, des Weiteren aufweisend die Schritte:
- Andocken der Vorrichtung (1) an die elektrische Zigarette, wodurch die Betätigungsvorrichtung (13a, 13b, 13c, 13d, 13e, 13f, 13g) freigegeben wird, und
- Abdocken der Vorrichtung (1) von der elektrischen Zigarette, wodurch die Betätigungsvorrichtung (13a, 13b, 13c, 13d, 13e, 13f, 13g) blockiert wird.

22. Verfahren zum Befüllen eines Verbrauchsmitteltanks (2) einer elektrischen Zigarette mittels einer Vorrichtung (1) zum Befüllen einer elektrischen Zigarette, aufweisend die Schritte:
- Fluidverbinden des Verbrauchsmitteltanks (2) mit einem ersten, mit einem Verbrauchsmittel gefüllten ersten Speicherraum (11),
- Fluidverbinden des Verbrauchsmitteltanks (2) mit einem zweiten Speicherraum (12),
- Volumenreduktion des ersten Speicherraums (11), sodass das Verbrauchsfluid von dem ersten Speicherraum (11) in den Verbrauchsmitteltank (2) gelangt, und
- Volumenvergrößerung des zweiten Speicherraums (12), sodass ein Ausgleichsfluid, insbesondere Luft, aus dem Verbrauchsmitteltank (2) in den zweiten Speicherraum (12) abgesaugt wird.

## Claims

1. Device (1) for filling an electric cigarette, comprising
a first storage space (11) for receiving a consumable substance,
an interface (14) with a first connection (141) which is fluidically connected to the first storage space (11), wherein the first connection is designed in such a way that it can be connected to the corresponding first connection of the consumable substance tank of an electric cigarette,
**characterised by**
a second storage space (12) for receiving a compensating fluid, wherein
the device (1) is designed in such a way that the volumes of the first storage space (11) and the second storage space (12) can be counteractively altered by means of an operating device (13a, 13b, 13c, 13d, 13e, 13f, 13g) and wherein the interface (14)
comprises a second connection (142) which is fludically connected to the second storage space (12) wherein the second connection is also designed in such a way that it can be connected to the corresponding second connection of the consumable substance tank of an electric cigarette.

2. Device (1) according to the preceding claim wherein
the first storage space (11) comprises a first hollow cylinder (111) and the second storage space (12) a second hollow cylinder (121), and
a volume change ΔV₁ of the first storage space (11) can be brought about by means of a first plunger (112) extendable and retractable into/out of the first hollow cylinder (111) and a volume change ΔV₂ of the second storage space (12) by means of second plunger (122) extendable and retractable into/out of the second hollow cylinder (121).

3. Device (1) according to any one of the preceding claims wherein
the operating device (13a, 13g) comprises a toothed wheel section (13a1),
the first plunger (112) and the second plunger (122) are directly or indirectly connected to each other by means of an operating rod (161) in the form of a toothed rod (161), threaded rod or other rod with tooth-like engagement elements and
the device (1) is designed in such a way that the toothed wheel section (13a1) and the operating rod (161) engage with each other.

4. Device (1) according to any one of claims 1 or 2 wherein
the operating device (13b) comprises a lever (13b2) wherein
the first plunger (112) and the second plunger (122) are directly or indirectly connected to the lever (13b2).

5. Device (1) according to the preceding claim wherein the
operating device (13b) comprises a pivot point (13b3) for the lever (13b3), wherein
the first plunger (112) and the second plunger (112) are directly or indirectly connected to each other by means of a rod (162b), wherein
the lever (13b2) and the rod (162b) are connected to each other.

6. Device (1) according to any one of claims 1 or 2 wherein
the operating device (13c) is connected in a non-rotational manner to a threaded rod (162c) wherein
the plungers (112, 122) have an inner thread and the device (1) is designed in such a way that the plungers (112, 122) are displaceable on the threaded rod (162c) relative to the first hollow cylinder (111) and the second hollow cylinder (121).

7. Device (1) according to any one of claims 1 or 2 wherein
the operating device (13d) has an inner thread, wherein
the first plunger (112) and the second plunger (122) are firmly connected to a threaded rod (162d), wherein
the device (1) is designed in such a way that by means of the operating device (13d) the threaded rood (162d) with the plunger (112, 122) is displaceable relative to the operating device (13d) and the hollow cylinders (111, 121).

8. Device (1) according to any one of claims 1 or 2 wherein
a rod (162e) is arranged non-rotationally with regard to an operating device (13e), but axially displaceably within the operating device (13e) wherein
the plungers (112, 122) are firmly connected to the rod (162e) and at least one of the hollow cylinders (111, 121) has an inner thread, wherein
at least one of the plungers (112, 122) engages in the inner thread of the at least one hollow cylinder (111, 112) by means of an outer thread or at least one pin, wherein
the device (1) is designed in such a way that by means of the operating device (13e) the rod (162e) with the plungers (112, 122) is displaceable relative to the cylinder (111, 121) and the operating device (13e) .

9. Device (1) according to claim 1 wherein
the first and the second storage space (11, 12) have a joint hollow cylinder (16f) and volume changes ΔV₁, ΔV₂ can be brought about by means of a plunger (15f) which is movable in the joint hollow cylinder (16f) .

10. Device (1) according to the preceding claim wherein
the operating device (13f) has a threaded rod (13f2) with an outer thread and the plunger (15f) an inner thread.

11. Device (1) according to any one of the preceding claims wherein
the device (1) is designed in such a way that in the storage spaces (11, 12) either volume changes ΔV1, ΔV2 of equal or unequal magnitude can be brought about.

12. Device (1) according to any one of the preceding claims wherein
the first connection (141) has a first cannula (14k) and the second connection (142) has a second cannula (14k).

13. Device (1) according to any one of the preceding claims also comprising a child-proof lock (18) which is provided with a latch (181, 182g) for blocking and releasing the operating device (13a, 13b, 13c, 13d, 13e, 13f, 13g).

14. Device (1) according to the preceding claim wherein the child-proof lock (18) is designed in such a way that it releases the operating device (13a, 13g) when the device (1) is in a release state and it is otherwise blocking the operating device (13a, 13g).

15. Device (1) according to any one of the preceding claims comprising a shell element which is provided to receive an electric cigarette and configured to define the position and alignment of an electric cigarette in one, two, three, four, five or six degrees of freedom.

16. Consumable substance tank (2) on an electric cigarette comprising
a first connection to be filled with consumable substance
**characterised in that**
the consumable substance tank (2) also comprises a second connection for releasing a compensating fluid, in particular air, wherein the first connection and the second connection are designed in such a way that they each can be connected to the corresponding first and second connection of the device according to claim 1.

17. Consumable substance tank (2) according to the preceding claim wherein
each of the connections (241, 242) comprises at least
one membrane (24m) or
one duckbill valve (24s) or
one ball valve (24k).

18. System (3) of a device (1) according to any one of the preceding claims 1 to 15 and a consumable substance tank (2) according to any of claims 16 to 17.

19. System (3) of a device (1) according to any one of the preceding claims 1 to 15 and a consumable substance tank (2) according to any of claims 16 to 17, wherein
the child-proof lock (18) is designed in such a way that this releases the operating device (13a, 13g) when the device (1) and the consumable substance tank (2) are in a filling position with regard to each other and the child-proof lock (18) is otherwise blocking the operating device (13a, 13g).

20. Method of filling a consumable substance tank (2) of an electric cigarette by means of a device (1) for filling an electric cigarette comprising the step:
- operating an operating device (13a, 13b, 13c, 13d, 13e, 13f, 13g) through which consumable substance is filled from the device (1) into the tank (2) of an electric cigarette
**characterised in that**
at the same time a compensating fluid volume displaced by the consumable substance being filled is drawn out of the tank (2) of the cigarette.

21. Method according to the preceding claims, also comprising the steps:
- docking of the device (1) on the electric cigarette, through which the operating device (13a, 13b, 13c, 13d, 13e, 13f, 13g) is released and
- undocking of the device (1) from the electric cigarette through which the operating device (13a, 13b, 13c, 13d, 13e, 13f, 13g) is blocked.

22. Method of filling a consumable substance tank (2) of an electric cigarette by means of a device (1) for filling an electric cigarette, comprising the steps:
- fluidic connection of the consumable substance tank (2) to a first storage space (11) filled with a consumable substance,
- fluidic connection of the consumable substance tank (2) to a second storage space (12),
- volume reduction of the first storage space (11) so that the consumable fluid moves from the first storage space (11) into the consumable substance tank (2) and
- volume enlargement of the second storage space (12) so that a compensating fluid, in particular air, is drawn from the consumable substance tank (2) into the second storage space (12).

## Revendications

1. Dispositif (1) de remplissage d'une cigarette électrique, comportant
un premier espace d'accumulation (11) pour recevoir une substance de consommation,
une interface (14) avec une première connexion (141), qui est reliée de manière fluidique au premier espace d'accumulation (11), la première connexion étant conçue de telle sorte qu'elle peut être raccordée à une première connexion correspondante du réservoir de produit de consommation d'une cigarette électrique,
**caractérisé par**
un deuxième espace d'accumulation (12) pour recevoir un fluide de compensation,
le dispositif (1) étant conçu de telle sorte que les volumes du premier espace d'accumulation (11) et du deuxième espace d'accumulation (12) peuvent être modifiés en sens contraire au moyen d'un dispositif d'actionnement (13a, 13b, 13c, 13d, 13e, 13f, 13g), et l'interface (14)
comportant une deuxième connexion (142), qui est reliée de manière fluidique au deuxième espace d'accumulation (12), la deuxième connexion étant également conçue de telle sorte qu'elle peut être raccordée à la deuxième connexion correspondante du réservoir de produit de consommation d'une cigarette électrique.

2. Dispositif (1) selon la revendication précédente, le premier espace d'accumulation (11) comportant un premier cylindre creux (111) et le deuxième espace d'accumulation (12) un deuxième cylindre creux (121), et
une modification de volume ΔV₁ du premier espace d'accumulation (11) pouvant être provoquée au moyen d'un premier piston (112) entrant et sortant dans le premier cylindre creux (111) et une modification de volume ΔV₂ du deuxième espace d'accumulation (12) pouvant être provoquée au moyen d'un deuxième piston (122) entrant et sortant dans le deuxième cylindre creux (121).

3. Dispositif (1) selon l'une quelconque des revendications précédentes, le dispositif d'actionnement (13a,13g) comportant une section de roue dentée (13a1),
le premier piston (112) et le deuxième piston (122) étant couplés indirectement ou directement l'un à l'autre au moyen d'une tige d'actionnement (161) sous la forme d'une crémaillère (161), d'une tige filetée ou d'une autre tige avec des éléments d'engagement dentés, et
le dispositif (1) étant conçu de sorte que la section à roue dentée (13a1) et la tige d'actionnement (161) viennent en prise l'une avec l'autre.

4. Dispositif (1) selon l'une quelconque des revendications 1 ou 2, le dispositif d'actionnement (13b) comportant un levier (13b2),
le premier piston (112) et le deuxième piston (122) étant couplés indirectement ou directement l'un à l'autre avec le levier (13b2).

5. Dispositif (1) selon l'une quelconque des revendications précédentes, le dispositif d'actionnement (13b) comportant un point de rotation (13b3) pour le levier (13b2),
le premier piston (112) et le deuxième piston (122) étant couplés indirectement ou directement l'un à l'autre au moyen d'une tige (162b),
le levier (13b2) et la tige (162b) étant couplés l'un à l'autre.

6. Dispositif (1) selon l'une quelconque des revendications 1 ou 2,
le dispositif d'actionnement (13c) étant relié solidaire en rotation à une tige filetée (162c),
les pistons (112,122) comportant un filetage intérieur, et
le dispositif (1) étant conçu de telle sorte que les pistons (112,122) peuvent être déplacés sur la tige filetée (162c) par rapport au premier cylindre creux (111) et au deuxième cylindre creux (121).

7. Dispositif (1) selon l'une quelconque des revendications 1 ou 2,
le dispositif d'actionnement (13d) comportant un filetage intérieur,
le premier piston (112) et le deuxième piston (122) étant reliés fermement à une tige filetée (162d),
le dispositif (1) étant conçu de telle sorte que la tige filetée (162d) peut être déplacée par rapport au dispositif d'actionnement (13d) et aux cylindres creux (111,121) au moyen du dispositif d'actionnement (13d).

8. Dispositif (1) selon l'une quelconque des revendications 1 ou 2,
une tige (162e) étant disposée solidaire en rotation par rapport à un dispositif d'actionnement (13e), mais axialement mobile à l'intérieur du dispositif d'actionnement (13e),
les pistons (112,122) étant reliés fermement à la tige (162e) et au moins un des cylindres creux (111, 121) comportant un filetage intérieur,
au moins un des pistons (112, 122) venant en prise dans le filetage intérieur d'au moins un cylindre creux (111,121) au moyen d'un filetage extérieur ou d'au moins un tourillon,
le dispositif (1) étant conçu de telle sorte que la tige (162e) peut être déplacée avec les pistons (112,122) par rapport aux cylindres (111,121) et au dispositif d'actionnement (13e) au moyen du dispositif d'actionnement (13e).

9. Dispositif (1) selon la revendication 1, le premier et le deuxième espaces d'accumulation (11,12) comportant un cylindre creux commun (16f) et des modifications de volume ΔV₁, ΔV₂ pouvant être provoquées au moyen d'un piston (15f) mobile dans le cylindre creux commun (16f) .

10. Dispositif (1) selon l'une quelconque des revendications précédentes,
le dispositif d'actionnement (13f) comportant une tige filetée (13f2) avec un filetage extérieur et le piston (15f) un filetage intérieur.

11. Dispositif (1) selon l'une quelconque des revendications précédentes,
le dispositif (1) étant conçu de telle sorte que des modifications de volume ΔV₁, ΔV₂ de valeur soit identique ou inégale peuvent être provoquées dans les espaces d'accumulation (11, 12).

12. Dispositif (1) selon l'une quelconque des revendications précédentes,
la première connexion (141) comportant une première canule (14k) et la deuxième connexion (142) une deuxième canule (14k).

13. Dispositif (1) selon l'une quelconque des revendications précédentes, comportant par ailleurs une sécurité enfant (18), qui est dotée d'un verrou (181,182g) pour bloquer et libérer le dispositif d'actionnement (13a,13b,13c,13d,13e,13f,13g).

14. Dispositif (1) selon la revendication précédente, la sécurité enfant (18) étant conçue de telle sorte que celle-ci libère le dispositif d'actionnement (13a,13g), lorsque le dispositif (1) se trouve dans un état de libération et bloque le dispositif d'actionnement (13a, 13g) dans le cas contraire.

15. Dispositif (1) selon l'une des revendications précédentes, comportant un élément à coque, qui est prévu pour recevoir une cigarette électrique et agencé à cet effet pour définir la position et l'orientation d'une cigarette électrique dans un, deux, trois, quatre, cinq ou six degrés de liberté.

16. Réservoir de produit de consommation (2) d'une cigarette électrique, comportant
une première connexion à remplir avec un produit de consommation,
**caractérisé en ce que**
le réservoir de produit de consommation (2) comporte en outre une deuxième connexion pour faire passer un fluide de compensation, notamment de l'air, la première connexion et la deuxième connexion étant conçues de telle sorte qu'elle peuvent être raccordées respectivement à une première et deuxième connexion correspondante du dispositif selon la revendication 1.

17. Réservoir de produit de consommation (2) selon la revendication précédente,
chacune des connexions (241,242) comportant au moins
une membrane (24m) ou
une soupape en bec de canard (24s) ou
une soupape sphérique (24k).

18. Système (3) d'un dispositif (1) selon l'une quelconque des revendications précédentes 1 à 15 et d'un réservoir de produit de consommation (2) selon l'une quelconque des revendications 16 à 17.

19. Système (3) d'un dispositif (1) selon l'une quelconque des revendications précédentes 13 à 15 et d'un réservoir de produit de consommation (2) selon l'une quelconque des revendications 16 à 17,
la sécurité enfant (18) étant conçue de telle sorte que celle-ci libère le dispositif d'actionnement (13a,13g), lorsque le dispositif (1) et le réservoir de produit de consommation (2) se trouvent dans une position de remplissage et la sécurité enfant (18) bloque dans le cas contraire le dispositif d'actionnement (13a,13g).

20. Procédé de remplissage d'un réservoir de produit de consommation (2) d'une cigarette électrique au moyen d'un dispositif (1) pour remplir une cigarette électrique, comportant les étapes de :
- actionnement d'un dispositif d'actionnement (13a,13b,13c,13d,13e,13f,13g), le produit de consommation étant introduit de ce fait du dispositif (1) dans le réservoir (2) d'une cigarette électrique,
**caractérisé en ce que**
simultanément un volume de fluide de compensation poussé par le produit de consommation introduit est aspiré du réservoir (2) de la cigarette.

21. Procédé selon l'une des revendications précédentes, comportant en outre les étapes de :
- accrochage du dispositif (1) à la cigarette électrique, le dispositif d'actionnement (13a,13b,13c,13d,13e,13f,13g) étant de ca fait libéré, et
- décrochage du dispositif (1) de la cigarette électrique, le dispositif d'actionnement (13a,13b,13c,13d,13e,13f,13g) étant de ce fait bloqué.

22. Procédé de remplissage d'un réservoir de produit de consommation (2) d'une cigarette électrique au moyen d'un dispositif (1) pour remplir une cigarette électrique, comportant les étapes de :
- raccordement de fluide du réservoir de produit de consommation (2) à un premier espace d'accumulation (11) rempli d'un produit de consommation,
- raccordement de fluide d'un réservoir de produit de consommation (2) à un deuxième espace d'accumulation (12),
- réduction de volume du premier espace d'accumulation (11) de telle sorte que le fluide de consommation parvient du premier espace d'accumulation (11) dans le réservoir de produit de consommation (2),
- augmentation de volume du deuxièmes espace d'accumulation (12) de telle sorte qu'un fluide de compensation, notamment de l'air, est aspiré du réservoir de produit de consommation (2) dans le deuxième espace d'accumulation (12).
